# FASCICULE DE BREVET EUROPEEN

(11) **EP 2 414 382 B1**
(45) Date de publication et mention de la délivrance du brevet: **12.07.2017**
(21) Numéro de dépôt: 10715913.9
(22) Date de dépôt: 01.04.2010
(51) Int. Cl.: C07K 7/06, C07K 5/113, A61K 8/64, A61Q 19/02, A61Q 19/08

(54) **PEPTIDES ECLAIRCISSANTS ACTIVATEURS DU PROTEASOME ET COMPOSITIONS LES CONTENANT**
AUFHELLEND WIRKENDE PEPTIDE ALS AKTIVATOREN DES PROTEASOMS UND SIE ENTHALTENDE ZUBEREITUNGEN
BLEACHING PEPTIDES FOR THE ACTIVATION OF THE PROTEASOME AND COMPOSITIONS COMPRISING THEM

(30) Priorité: 02.04.2009 FR 0901613
(43) Date de publication de la demande: 08.02.2012
(73) Titulaire: ISP Investments Inc., Wilmington, DE 19805 (US)
(72) Inventeur: DAL FARRA, Claude, Kerhonkson New York 12446 (US); DOMLOGE, Nouha, F- 06560 Valbonne (FR); BOTTO, Jean-Marie, F-06560 Valbonne (FR)
(74) Mandataire: Macquet, Christophe
(86) Numéro de dépôt international: PCT/FR2010/000278
(87) Numéro de publication internationale: WO 2010/112711

(56) Documents cités:
- WO-A1-2005/061530
- WO-A1-2007/131774

### SEQUENCE LISTING

<110> ISP Investments Inc
<120> NOUVEAUX PEPTIDES ECLAIRCISSANTS ACTIVATEURS DU PROTEASOME ET COMPOSITIONS LES CONTENANT
<130> Bv 09-119
<150> FR 09 01613
   <151> 2009-04-02
<160> 5
<170> patentIn version 3.3
<210> 1
   <211> 5
   <212> PRT
   <213> Artificial
<220>
   <223> synthetic peptide
<400> 1
   Arg Asp Cys Arg Arg
   1               5
<210> 2
   <211> 3
   <212> PRT
   <213> Artificial
<220>
   <223> Synthetic peptide
<220>
   <221> MOD_RES
   <222> (3)..(3)
   <223> AMIDATION
<400> 2
   Asp Cys Arg
   1
<210> 3
   <211> 7
   <212> PRT
   <213> Artificial
<220>
   <223> Synthetic peptide
<220>
   <221> MOD_RES
   <222> (7)..(7)
   <223> AMIDATION
<400> 3
   Ser Arg Asp Cys Arg Pro Met
   1               5
<210> 4
   <211> 6
   <212> PRT
   <213> Artificial
<220>
   <223> Synthetic peptide
<400> 4
   Thr Asp Cys Arg Lys Arg
   1               5
<210> 5
   <211> 7
   <212> PRT
   <213> Artificial
<220>
   <223> Synthetic peptide
<220>
   <221> MOD_RES
   <222> (7)..(7)
   <223> AMIDATION
<400> 5
   Asp Cys Arg Arg Pro Met Gly
   1               5

## Description

La présente invention se rapporte à des composés peptidiques de formule générale R₁- X₁ - X₂ -Asp - Cys - Arg - X₃ -X₄ - (AA)p - R₂ en tant qu'agents éclaircissants, dépigmentants et/ou blanchissants, ainsi que leurs utilisations en cosmétique et/ou pharmaceutique en tant qu'agents éclaircissants, dépigmentants et/ou blanchissants. Lesdits composés permettent en outre de prévenir et/ou traiter les signes cutanés d'ordre hyperpigmentaires dus au photo-vieillissement. Les compositions comprenant lesdits composés peptidiques permettent, enfin, de traiter les taches d'hyperpigmentation d'origines diverses.

Chez l'humain, la couleur des cheveux et de la peau est liée à des facteurs individuels (origine ethnique, sexe, âge, etc.) et à des facteurs environnementaux (notamment les saisons de l'année, la zone d'habitation, etc.). Elle est principalement déterminée par la nature et la concentration de mélanine produite par les mélanocytes. La mélanine a la propriété de protéger les cellules cutanées des effets délétères des rayonnements UV et de ralentir le photo-vieillissement cutané. Les mélanocytes sont les cellules spécialisées qui, par l'intermédiaire d'organites particuliers, les mélanosomes, synthétisent la mélanine. La synthèse de la mélanine ou mélanogénèse est un processus complexe dont les mécanismes précis ne sont pas encore élucidés et qui fait intervenir schématiquement les étapes suivantes :
Tyrosine→ Dopa → Dopaquinone → Dopachrome →Mélanine.

Cette mélanine joue en effet un rôle fondamental dans la détermination de la couleur de la peau. Elle est synthétisée par de larges cellules dendritiques : les mélanocytes, cellules localisées dans la couche basale de l'épiderme. La mélanine existe sous deux formes différentes : phaeomélanine, pigment de couleur jaune, et l'eumélanine de couleur noire. Ce sont les différentes proportions et la taille de ces pigments, sans oublier les caroténoïdes et la micro-circulation sanguine, qui donnent à la peau sa grande diversité de couleur.

La production de mélanine, ainsi que son transport, sont régulés par différents facteurs tels que, par exemple, le rayonnement UV, les hormones ou les produits chimiques. Ainsi, une augmentation de l'exposition aux rayonnements UV provoque la synthèse de pigments et résulte en un assombrissement de la peau. Des perturbations de cette pigmentation, plus ou moins bénignes, peuvent apparaître. Elles se manifestent, par exemple, par des taches de rousseur, des grains de beauté, des taches diffuses telles que les taches de grossesse, le chloasma, le mélasma, ainsi que par d'autres désordres hyperpigmentaires comme par exemple le lentigo. D'autres taches d'hyperpigmentation peuvent être dues par exemple à une mauvaise cicatrisation notamment chez les individus de phénotype foncé, ou encore elles peuvent être dues à l'utilisation de médicaments photo-sensibilisants etc. Mais le vieillissement peut lui aussi moduler la pigmentation cutanée. Ainsi, certaines personnes peuvent voir apparaître des taches sur la peau, plus ou moins foncées ou colorées, conférant des zones de colorations hétérogènes formant des taches de sénescence ou encore des éphélides. Enfin, dans certaines populations de type asiatique ou encore africaine, des soins cosmétiques éclaircissants sont recherchés afin d'obtenir un teint clair et uniforme.

L'utilisation d'inhibiteur ou de régulateur de la synthèse de mélanine, ainsi que de tout autre produit dépigmentant et/ou blanchissant, est donc particulièrement intéressant en cosmétologie et/ou en dermatologie. Cette utilisation est non seulement intéressante lorsqu'une véritable dépigmentation de la peau est recherchée, comme dans le cas de blanchiment de peau fortement pigmentée ou encore lors d'inhibition de zones cutanées hyperpigmentées résultant en un aspect inesthétique de la peau ; elle l'est également dans certaines applications visant à éclaircir le teint, à donner de la luminosité à la peau ou encore à donner de l'éclat aux tissus de surface.

Jusqu'à ce jour, de nombreuses molécules ont été proposées avec plus ou moins d'efficacité. Parmi ces molécules, on peut citer des dérivés du phénol tels que l'hydroquinone et le résorcinol qui inhibent une série de réaction de la conversion de la L- tyrosine en mélanine par inhibition de l'activité de la tyrosinase (Takano, 1984). On peut citer aussi l'acide L-ascorbique et ses dérivés, le magnésium ascorbyl acétate, l'acide kojique ou encore l'acide lactique.

Mais la plupart des produits actuellement sur le marché sont toxiques et/ou ne présentent pas une efficacité suffisante. Par exemple, l'hydroquinone est irritante et cytotoxique pour le mélanocyte. L'acide kojique par exemple n'est pas stable en solution, etc. Il existe donc un besoin pour un nouvel agent blanchissant qui ne présenterait pas les inconvénients de ceux existants mais qui serait tout aussi efficace. C'est ainsi que d'autres voies ont été explorées afin de trouver un nouvel agent dépigmentant ayant une action à la fois sur l'enzyme tyrosinase et sur la mélanine.

De manière surprenante, la Demanderesse a découvert que des composés peptidiques de formule R₁- X₁- X₂ -Asp - Cys - Arg - X₃ -X₄ - (AA)ₚ - R₂ étaient de très bons agents blanchissants et présentaient une bonne activité dépigmentante sans pour autant être toxique. Ces composés peptidiques sont en fait des composés activateurs du protéasome.

La voie ubiquitine-protéasome joue un rôle fondamental dans un très grand nombre de processus biologiques. En effet, les mécanismes de dégradation des protéines par le protéasome sont impliqués dans des mécanismes cellulaires importants tels que la réparation de l'ADN, le contrôle de l'expression des gènes, la régulation de la progression du cycle cellulaire, le contrôle de la qualité des protéines néosynthétisées, l'apoptose ou la réponse immunitaire (Glickman and Ciechanover, 2002).

Le protéasome présent dans les cellules humaines est un complexe multi protéique de très grande taille présent dans le cytoplasme et le noyau. Les formes purifiées du protéasome comprennent 2 grandes sous-unités ; d'une part, un coeur protéolytique appelé protéasome 2OS et, d'autre part, un complexe régulateur 19S qui se lie à chacune des deux extrémités du protéasome 2OS (Coux et *al.,* 1996 ; Glickman et Coux, 2001). Le protéasome 2OS est une particule en forme de cylindre creux, composée de 28 sous-unités alpha et béta, distribuées en 4 anneaux heptamériques. Les activités peptidases sont présentes à la surface interne du cylindre et s'influencent de manière allostérique. Trois activités protéolytiques (« trypsine, chymotrypsine et caspase-like ») ont été associées au protéasome 2OS et concourent à la destruction des protéines en peptides inactifs de 3 à 20 acides aminés. Outre le protéasome 2OS le protéasome 26S comprend le complexe régulateur 19S de 0,7 MDa constitué de 20 sous-unités environ. Des études récentes d'immunopurification ont montré que d'autres protéines pouvaient être associées au protéasome 20S et 19S (par exemple le complexe régulateur 11S).

Au vu de la diversité des processus cellulaires contrôlés via la dégradation des protéines, il n'est pas surprenant de constater que des altérations de la voie ubiquitine-protéasome sont à l'origine, ou étroitement liées à plusieurs maladies génétiques et à de nombreuses pathologies humaines telles que les cancers colorectaux, les lymphomes, les syndromes inflammatoires, les maladies neuro-dégénératives telles que Parkinson ou Alzheimer...

De nombreux travaux ont été menés ces dernières années sur le rôle du protéasome dans le vieillissement de la peau. Une des voies explorées dernièrement se tournait vers le protéasome et son action sur la dégradation des protéines impliquées dans la mélanogénèse. Des expériences ont montrées que la dégradation de l'enzyme tyrosinase par le protéasome était activée par un extrait d'algues sur des mélanocytes humains (Nizard et al. Antioxid. Redox Signal ; 2006, 1-2 : 136-143).

Une composition comprenant un extrait de silybine, de *Bletilla striata* et *d'Iris sanguinea* capable d'augmenter l'activité du protéasome et permettant d'avoir un effet sur la pigmentation de la peau a été divulguée dans une demande de brevet (US-A1-2009041866). WO-A1-2005061530 (CNRS) fait état de différents activateurs des protéasomes de nature peptidique.

C'est ainsi que la Demanderesse a découvert que des composés de formule (I) R₁- X₁- X₂ -Asp - Cys - Arg - X₃ -X₄ - (AA)ₚ - R₂ étaient capables d'activer le protéasome et pouvaient ainsi dépigmenter, éclaircir ou encore blanchir la peau et les phanères.

Par conséquent la présente invention a pour premier objet un composé peptidique caractérisé en ce qu'il correspond à l'une des formules suivantes :
(SEQ ID n° 1) Arg - Asp -Cys -Arg - Arg
(SEQ ID n° 2) Asp -Cys -Arg - NH2
(SEO ID n° 3) Ser -Arg - Asp - Cys -Arg - Pro - Met - NH2
(SEO ID n° 4) Thr - Asp - Cys -Arg - Lys - Arg
(SEO ID n° 5) Asp - Cys - Arg - Pro - Met - Gly - NH2;
lesdites séquences de formules SEQ ID n°1 à SECS ID n°5 pouvant comprendre des substitutions entre les résidus :
- Ser et Thr ;
- Asp et Glu ;
- Lys et Arg.

L'invention a pour second objet une composition cosmétique comprenant à titre de principe actif ledit composé.

L'invention a pour troisième objet l'utilisation d'une composition cosmétique comprenant :
- un composé peptidique de formule générale suivante (I) :
   R₁- X₁- X₂ -Asp - Cys - Arg - X₃ -X₄ - (AA)ₚ- R₂

Dans laquelle,
X₁ représente un acide aspartique, un acide glutamique, une sérine, une thréonine, ou est égal à zéro,
X₂ représente une arginine, une leucine, une isoleucine, ou est égal à zéro,
X₃ représente une arginine, une lysine, ou est égal à zéro,
X₄ représente une arginine, une proline, une histidine, une lysine, ou est égal à zéro,
AA représente un acide aminé quelconque à l'exception de la cystéine, ou un de ses dérivés, et p est un nombre entier compris entre 0 et 2,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 3 à 9 résidus d'acides aminés,
ladite séquence de formule générale (I) pouvant comprendre des substitutions des acides aminés X₁, X₂, X₃, et X₄ par d'autres acides aminés chimiquement équivalents, et
un milieu cosmétiquement acceptable,
pour dépigmenter, éclaircir et/ou blanchir la peau.

En outre, la présente invention a pour quatrième objet l'utilisation d'une composition cosmétique comprenant le composé peptidique tel que défini dans le premier objet de l'invention et un milieu cosmétiquement acceptable pour dépigmenter, éclaircir et/ou blanchir la peau.

La présente invention a pour cinquième objet un procédé de traitement cosmétique caractérisé en ce que l'on applique topiquement sur la peau ou les phanères à traiter, une composition comprenant une quantité efficace de composé peptidique à titre de principe actif, de formule générale (I), pour dépigmenter, éclaircir et/ou blanchir la peau.

Enfin, la présente invention a pour sixième objet un procédé de traitement cosmétique caractérisé en ce que l'on applique topiq_uement sur la peau ou les phanères à traiter, une composition comprenant une quantité efficace de composé peptidique à titre de principe actif tel que défini dans le premier objet de l'invention pour dépigmenter, éclaircir et/ou blanchir la peau ou les phanères.

La présente invention a pour objet une composition comprenant un composé peptidique activateur du protéasome de formule générale suivante (I) :
R₁- X₁ - X₂ -Asp - Cys - Arg - X₃ -X₄ - (AA)ₚ- R₂

Dans laquelle,
X₁ représente un acide aspartique, un acide glutamique, une sérine, une thréonine, ou est égal à zéro,
X₂ représente une arginine, une leucine, une isoleucine, ou est égal à zéro,
X₃ représente une arginine, une lysine, ou est égal à zéro,
X₄ représente une arginine, une proline, une histidine, une lysine, ou est égal à zéro,
AA représente un acide aminé quelconque à l'exception de la cystéine, ou un de ses dérivés, et p est un nombre entier compris entre 0 et 2,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 3 à 9 résidus d'acides aminés,
ladite séquence de formule générale (I) pouvant comprendre des substitutions des acides aminés X₁ X₂, X₃, et X₄ par d'autres acides aminés chimiquement équivalents,
On entend par composé peptidique « activateur du protéasome», tout peptide ou dérivé biologiquement actif capable d'augmenter l'activité du protéasome, soit par augmentation de la synthèse protéique des sous-unités du protéasome (par modulation directe ou indirecte de l'expression génique) soit par d'autres processus biologiques tels que la stabilisation des sous-unités constituant le protéasome ou encore la stabilisation des transcrits d'ARN messager.

Le composé peptidique selon l'invention est caractérisé en ce qu'il permet d'activer la dégradation par le protéasome des protéines endommagées impliquées dans la mélanogénèse. Par « protéines endommagées » on entend des protéines ayant subi des réactions d'oxydation dues aux espèces réactives de l'oxygène (radicaux libres), des protéines glyquées ou conjuguées avec des produits issus de la péroxydation lipidique, etc. Par « impliquées dans la mélanogénèse » on entend toute protéine participant directement ou indirectement à la synthèse de mélanine, telle que l'enzyme tyrosinase, ou encore la mélanine elle-même.

Dans un premier mode de réalisation préféré, le composé peptidique est protégé par une acylation ou par une acétylation de l'extrémité C-terminale.

Dans un second mode de réalisation préféré, le composé peptidique correspond à une des formules suivantes :
(SEQ ID n° 1) Arg - Asp -Cys -Arg - Arg
(SEQ ID n°2) Asp - Cys -Arg - NH₂
(SEQ ID n°3) Ser -Arg - Asp - Cys -Arg - Pro - Met - NH₂
(SEQ ID n°4) Thr - Asp - Cys -Arg - Lys - Arg
(SE ID n°5) Asp - Cys -Arg - Arg - Pro - Met - Gly - NH₂

Préférentiellement, le composé peptidique selon l'invention correspond à la séquence ID n° c'est-à-dire Arg - Asp -Cys -Arg - Arg.

Dans un autre mode de réalisation préféré, le composé peptidique selon l'invention correspond à la séquence ID n°2 c'est-à-dire Asp -Cys -Arg - NH₂.

L'invention concerne aussi des formes homologues de ces séquences. Le terme « homologue » désigne, selon l'invention, toute séquence peptidique identique à au moins 50%, ou de préférence au moins 80%, et encore plus préférentiellement à au moins 90% de ladite séquence peptidique, choisie parmi les séquences SEQ ID n° 1 à SEQ ID n° 5. Par «séquence peptidique identique à au moins X% », on entend désigner un pourcentage d'identité entre les résidus d'acides aminés des deux séquences à comparer, obtenu après l'alignement optimal des deux séquences. L'alignement optimal est obtenu à l'aide d'algorithmes d'homologies locales tels que ceux utilisés par le logiciel informatique BLAST P disponible sur le site NCBI.

Le terme « homologue » peut également désigner un peptide qui diffère de la séquence d'un peptide de séquence SEQ ID n° 1 à SEQ ID n° 5 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et IIe ; entre Ser et Thr ; entre les résidus acides Asp et Glu ; entre Asn et Gln ; et entre les résidus basiques Lys et Arg ; ou entre les résidus aromatiques Phe et Tyr.

Les acides aminés constituant le peptide selon l'invention peuvent être sous configuration Lévogyre c'est-à-dire L- et/ou Dextrogyre c'est-à-dire D-. Le peptide selon l'invention peut donc être sous forme L-, D- ou DL-.

Le terme « peptide » ou « composé peptidique » désigne un enchaînement de deux ou plusieurs acides aminés liés entre eux par des liaisons peptidiques ou par des liaisons peptidiques modifiées. Ce terme est équivalent au terme « principe actif » qui sera utilisé également.

Par « peptide » ou « composé peptidique », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées. Elles sont bien connues de l'homme du métier comme, par exemple, l'acylation ou l'acétylation des extrémités amino et/ou carboxy-terminales. Ainsi, l'invention concerne une composition telle que définie précédemment, caractérisée par le fait que le peptide de séquence SEQ ID n°1à SEQ ID n°5 est sous forme protégée de façon simple ou double. De préférence, on utilise, pour la protection de la fonction hydroxyle de l'extrémité carboxy-terminale, une amidation par un groupement NYY avec Y représentant une chaîne alkyle de C₁ à C₄, ou l'estérification par un groupement alkyle. Il est également possible de protéger les deux extrémités du peptide.

Le peptide de formule générale (I) selon l'invention peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide selon l'invention peut être d'origine naturelle ou synthétique. Préférentiellement selon l'invention, le peptide est obtenu par synthèse chimique.

Enfin, le principe actif peut être un peptide unique, un mélange de peptides ou de dérivés peptidiques et/ou constitués de dérivés d'acides aminés.

Le composé peptidique selon l'invention peut être utilisé à titre de médicament.

Le second objet de la présente invention se rapporte à des compositions cosmétiques comprenant ledit composé peptidique de formule générale (I) à titre de principe actif. De manière préférée, les compositions selon l'invention se présentent sous une forme adaptée à l'application par voie topique comprenant un milieu cosmétiquement acceptable. Par « cosmétiquement acceptable », on entend des milieux qui conviennent à une utilisation en contact avec la peau ou les phanères humains, sans risque de toxicité, d'incompatibilité, d'instabilité, de réponse allergique et autres. Préférentiellement, ledit composé peptidique est présent dans la composition à une concentration comprise entre 0,0005 et 500 ppm environ, et préférentiellement à une concentration comprise entre 0,01 et 5 ppm. Dans les compositions selon l'invention, le composé peptidique est solubilisé dans un ou plusieurs solvants comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux, le principe actif selon l'invention est solubilisé dans un vecteur comme les liposomes ou adsorbé sur des polymères organiques poudreux, des supports minéraux comme les talcs et bentonites, et plus généralement solubilisé dans, ou fixé sur, tout vecteur physiologiquement acceptable.

Les compositions destinées à être appliquées sur la peau peuvent se présenter sous forme de solution aqueuse ou hydro-alcoolique, d'émulsion eau dans huile ou huile dans eau, de microémulsion, de gel aqueux ou anhydre, de sérum, ou encore de dispersion de vésicules, de patch, de crème, de spray, d'onguent, de pommade, de lotion, de colloïde, de solution, de suspension ou autres. Les compositions peuvent aussi être appliquées sur les phanères sous forme de shampoing, de teinture ou de mascara à appliquer au pinceau ou au peigne, en particulier sur les cils, les sourcils ou les cheveux, ou encore de soins pour les ongles tels que les vernis.

Dans un mode de réalisation particulier, la composition selon l'invention contient en outre, au moins un autre principe actif favorisant l'action dudit composé peptidique. On peut citer, de manière non limitative, les classes d'ingrédients présentant une activité dans le domaine des agents éclaircissants tels que des agents desquamants; des agents apaisants, des agents photo-protecteurs organiques ou inorganiques, des agents hydratants; d'autres agents dépigmentants, des inhibiteurs de tyrosinase; des agents stimulant la synthèse de macromolécules dermiques ou épidermiques et/ou empêchant leur dégradation; des agents stimulant la prolifération des fibroblastes et/ou des kératinocytes ou stimulant la différenciation des kératinocytes; des agents agissant sur le métabolisme énergétique des cellules; d'autres peptides dépigmentants, des hydrolysats végétaux, des agents anti-âge, ainsi que leurs mélanges. En outre, des additifs tels que des agents épaississants, émulsifiants, humectants, émollients, des parfums, des antioxydants, des agents filmogènes, chélatants, séquestrants, conditionnants... peuvent être ajoutés à la composition.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être compris entre 0,01 et 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Enfin, la composition telle que décrite permet d'augmenter l'activité du protéasome et d'améliorer la dégradation par le protéasome des protéines endommagées impliquées dans la mélanogènese.

Un troisième objet de l'invention se rapporte à l'utilisation d'une composition cosmétique comprenant ledit composé peptidique et un milieu cosmétiquement acceptable pour dépigmenter, éclaircir et/ou blanchir la peau.

La composition selon l'invention permet aussi de prévenir et/ou traiter les taches d'hyperpigmentation telles que le mélasma, le chloasma, le lentigo actinique, les lentigos solaires, les éphélides, les taches d'hyperpigmentation accidentelles, les taches d'hyperpigmentation post- cicatricielles.

Un autre objet de l'invention se rapporte à l'utilisation d'une composition comprenant ledit composé peptidique pour traiter et/ou prévenir les signes cutanés d'ordre hyperpigmentaire dus au photo-vieillissement. Par « photo-vieillissement » on entend le vieillissement prématuré de la peau causé par l'exposition prolongée et cumulative au soleil.

Enfin, un autre objet de l'invention se rapporte à l'utilisation d'une composition cosmétique selon l'invention pour augmenter l'activité du protéasome et améliorer la dégradation par le protéasome des protéines endommagées impliquées dans la mélanogènese.

Un dernier objet de la présente invention se rapporte à un procédé de traitement cosmétique caractérisé en ce que l'on applique topiquement sur la peau ou les phanères à traiter, une composition comprenant une quantité efficace de composé peptidique selon l'invention pour dépigmenter, éclaircir et/ou blanchir la peau ou les phanères. Par ailleurs, ce procédé de traitement cosmétique est aussi destiné à prévenir et/ou traiter les signes cutanés d'ordre hyperpigmentaire dus au photovieillissement.

Les exemples suivants décrivent et démontrent l'efficacité de composés peptidiques tels que décrits selon l'invention. La formulation cosmétique citée est représentative de l'invention mais est donnée uniquement à titre d'illustration et ne doit pas être interprétée comme une limitation de la présente invention.

### Exemple 1 : Mise en évidence de l'effet dépigmentant de l'actif par des tests ex vivo.

L'activité dépigmentante de l'actif de séquence ID n°2 a été mise en évidence sur un échantillon de peau.

Des biopsies de 6 mm de diamètre sont prélevées sur des échantillons de peau humaine. Ces biopsies sont maintenues en survie *ex vivo* par culture en présence d'un milieu spécifique (DMEM 1 g/L, HAMF12, SVF et antibiotiques) sur des inserts déposés dans des plaques 6 puits. Les biopsies sont alors prétraitées pendant 24 heures avec l'actif à la concentration de 3 % à raison de 2 applications par jour. D'autres échantillons de peau ne seront pas prétraités avec l'actif et serviront de condition contrôle. Par la suite, les biopsies sont soumises à des irradiations UVB à raison de 100 mJ/cm² Les biopsies prétraitées sont traitées à nouveau pendant 24 heures avec l'actif à la concentration de 3 % à raison de 2 applications par jour. Après ce second traitement, une évaluation quantitative de la quantité de mélanine présente dans l'épiderme des échantillons de peau est réalisée histologiquement, au microscope optique, après une coloration suivant la méthode de Fontana-Masson.

Pour cela, les biopsies de peau sont incluses dans la paraffine et des coupes histologiques de 4 µm d'épaisseur sont effectuées. Ces coupes sont alors colorées par la technique de Fontana-Masson : les lames sont déparaffinées, hydratées puis traitées avec une solution d'ammoniaque d'argent. Après un passage de deux minutes au micro-onde, les lames sont rincées, traitées avec du sodium thiosulfate, rincées à nouveau et contre-colorées avec de l'hématoxyline avant d'être déshydratées et montées sous lamelles permettant ainsi la visualisation au microscope optique de la mélanine présente dans l'épiderme.

### Résultats :

Avant irradiation UVB, les peaux non traitées présentent un niveau de pigmentation plus élevé comparé aux peaux traitées avec l'actif. Après irradiation UVB, les peaux traitées avec l'actif présentent un niveau de pigmentation nettement inférieur comparé aux peaux non traitées de la condition contrôle. Par conséquent, ces résultats nous permettent de conclure qu'en l'absence d'une irradiation par les UVB, l'actif diminue le taux de mélanine par comparaison avec l'échantillon de peau non traitée. Par ailleurs, la synthèse de mélanine, induite par les irradiations aux UVB, est très nettement atténuée lorsque les échantillons de peaux sont prétraités avec l'actif. Ainsi, l'actif selon l'invention permet de diminuer significativement la pigmentation de la peau et permet d'obtenir une inhibition de la synthèse de mélanine.

### Exemple 2 : Composition cosmétique dépigmentante avec filtre solaire

| ***Noms commerciaux*** | ***Noms INCI*** | ***% massique*** |
|---|---|---|
| ***PHASE A*** | | |
| Montanov 68 | Cetearyl Alcohol (and) Cetearyl Glucoside | 5.00 |
| Isopropyl Palmitate | Isopropyl Palmitate | 7.00 |
| Waglinol 250 | Cetearyl Ethylhexanoate | 3.00 |
| Dow Corning 200 | Dimethicone Polydimethylsiloxane | 0.50 |
| Parsol MCX | Ethylhexyl Methoxycinnamate | 3.00 |
| Parasol 1789 | Butyl Methoxydibenzoylmethane | 1.00 |
| Phenonip | Phenoxyethanol (and) Methylparaben (and) Ethylparaben (and) Butylparaben (and) Propylparaben (and) Isobutylparaben | 0.50 |
| Cegesoft PS6 | Vegetable Oil | 2.00 |
| Huile de Jojoba | Simmondsia Chinensis (Jojoba) Seed Oil | 5.00 |

| ***PHASE B*** | | |
|---|---|---|
| Eau Demineralisee | Aqua (Water) | qsp |
| Glycerine | Glycerin | 3.00 |
| Glucam E10 | Methyl Gluceth-10 | 0.50 |
| EDTA Tetrasodium | EDTA | 0.20 |

| ***Noms commerciaux*** | ***Noms INCI*** | ***% massique*** |
|---|---|---|
| ***PHASE C*** | | |
| Sepigel 305 | Polyacrylamide (and) C13-14 Isoparaffin (and) Laureth-7 | 0.35 |
| Jus de Citron | Citrus medica Limonum (Lemon) Fruit Extract | 0.23 |

| ***PHASE D*** | | |
|---|---|---|
| Peptide SEQ ID n°5 | | 1.50 |
| Compo parfumante | Parfum (Fragrance) | qsp |
| Colorant | Dye | qsp |

## Revendications

1. Composé peptidique **caractérisé en ce qu'**il correspond à une des formules suivantes :
(SEQ ID n° 1) Arg - Asp -Cys -Arg - Arg
(SEQ ID n° 2) Asp -Cys -Arg - NH2
(SEQ ID n° 3) Ser -Arg - Asp - Cys -Arg - Pro - Met - NH2
(SEQ ID n° 4) Thr - Asp - Cys -Arg - Lys - Arg
(SEQ ID n° 5) Asp - Cys -Arg - Arg - Pro - Met - Gly - NH2;
lesdites séquences de formules SEQ ID n°1 à SEQ ID n°5 pouvant comprendre des substitutions entre les résidus :
- Ala, Val, Leu et Ile ;
- Ser et Thr ;
- Asp et Glu ;
- Asn et Gln ;
- Lys et Arg ; et
- Phe et Tyr.

2. Composé peptidique selon la revendication 1, **caractérisé en ce qu'**il correspond à une des formules suivantes :
(SEQ ID n°1) Arg - Asp -Cys -Arg - Arg
(SEQ ID n°2) Asp -Cys -Arg - NH₂
(SEQ ID n°3) Ser -Arg - Asp - Cys -Arg - Pro - Met - NH₂
(SEQ ID n°4) Thr - Asp - Cys -Arg - Lys - Arg
(SEQ ID n°5) Asp - Cys -Arg - Arg - Pro - Met - Gly - NH₂

3. Composé peptidique selon l'une des revendications précédentes, **caractérisé en ce qu'**il est utilisé à titre de médicament.

4. Composition cosmétique comprenant à titre de principe actif ledit composé peptidique selon l'une des revendications 1 ou 2.

5. Composition cosmétique selon la revendication 4, **caractérisée en ce qu'**elle se présente sous une forme adaptée à l'application par voie topique comprenant un milieu cosmétiquement acceptable.

6. Composition selon l'une des revendications 4 ou 5, **caractérisée en ce que** ledit composé peptidique est présent dans la composition à une concentration comprise entre 0,0005 et 500 ppm environ, et préférentiellement à une concentration comprise entre 0,01 et 5 ppm.

7. Composition selon l'une quelconque des revendications 4 à 6, **caractérisée en ce qu'**elle contient en outre, au moins un autre principe actif favorisant l'action dudit composé peptidique.

8. Composition selon la revendication 7, **caractérisée en ce que** ledit principe actif est un agent présentant une activité dans le domaine des agents éclaircissants tels que des agents desquamants; des agents apaisants, des agents photo-protecteurs organiques ou inorganiques, des agents hydratants; d'autres agents dépigmentants, des inhibiteurs de tyrosinase; des agents stimulant la synthèse de macromolécules dermiques ou épidermiques et/ou empêchant leur dégradation; des agents stimulant la prolifération des fibroblastes et/ou des kératinocytes ou stimulant la différenciation des kératinocytes; des agents agissant sur le métabolisme énergétique des cellules; d'autres peptides dépigmentants, des hydrolysats végétaux, des agents anti-âge, ainsi que leurs mélanges.

9. Utilisation d'une composition cosmétique comprenant :
- un composé peptidique de formule générale suivante (I) :
R₁ - X₁ - X₂ -Asp - Cys - Arg - X₃ -X₄ - (AA)ₚ- R₂
dans laquelle,
X₁ représente un acide aspartique, un acide glutamique, une sérine, une thréonine, ou est égal à zéro,
X₂ représente une arginine, une leucine, une isoleucine, ou est égal à zéro,
X₃ représente une arginine, une lysine, ou est égal à zéro,
X₄ représente une arginine, une proline, une histidine, une lysine, ou est égal à zéro,
AA représente un acide aminé quelconque à l'exception de la cystéine, ou un de ses dérivés, et p est un nombre entier compris entre 0 et 2,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 3 à 9 résidus d'acides aminés,
ladite séquence de formule générale (I) pouvant comprendre des substitutions des acides aminés X₁, X₂, X₃, et X₄ par d'autres acides aminés chimiquement équivalents ; et
- un milieu cosmétiquement acceptable,
pour dépigmenter, éclaircir et/ou blanchir la peau.

10. Utilisation d'une composition cosmétique comprenant ledit composé peptidique tel que défini dans l'une des revendications 1 ou 2 et un milieu cosmétiquement acceptable pour dépigmenter, éclaircir et/ou blanchir la peau.

11. Utilisation d'une composition selon l'une des revendications 9 ou 10, pour prévenir et/ou traiter les taches d'hyperpigmentation telles que le mélasma, le chloasma, le lentigo actinique, les lentigos solaires, les éphélides, les taches d'hyperpigmentation accidentelles, les taches d'hyperpigmentation post-cicatricielles.

12. Utilisation d'une composition selon l'une des revendications 9 ou 10, pour traiter et/ou prévenir les signes cutanés d'ordre hyperpigmentaire dus au photo-vieillissement.

13. Procédé de traitement cosmétique **caractérisé en ce que** l'on applique topiquement sur la peau ou les phanères à traiter, une composition comprenant une quantité efficace de composé peptidique à titre de principe actif, de formule générale suivante (1) :
R₁ - X₁ - X₂ -Asp - Cys - Arg - X₃ -X₄ - (AA)ₚ - R₂
dans laquelle,
X₁ représente un acide aspartique, un acide glutamique, une sérine, une thréonine, ou est égal à zéro,
X₂ représente une arginine, une leucine, une isoleucine, ou est égal à zéro,
X₃ représente une arginine, une lysine, ou est égal à zéro,
X₄ représente une arginine, une proline, une histidine, une lysine, ou est égal à zéro,
AA représente un acide aminé quelconque à l'exception de la cystéine, ou un de ses dérivés, et p est un nombre entier compris entre 0 et 2,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 3 à 9 résidus d'acides aminés,
ladite séquence de formule générale (I) pouvant comprendre des substitutions des acides aminés X₁, X₂, X₃, et X₄ par d'autres acides aminés chimiquement équivalents ;
pour dépigmenter, éclaircir et/ou blanchir la peau.

14. Procédé de traitement cosmétique **caractérisé en ce que** l'on applique topiquement sur la peau ou les phanères à traiter, une composition comprenant une quantité efficace de composé peptidique à titre de principe actif tel que défini dans l'une des revendications 1 ou 2, pour dépigmenter, éclaircir et/ou blanchir la peau ou les phanères.

15. Procédé de traitement cosmétique, tel que défini dans l'une des revendications 13 ou 14, pour prévenir et/ou traiter les signes cutanés d'ordre hyperpigmentaire dus au photovieillissement.

## Patentansprüche

1. Peptidverbindung, **dadurch gekennzeichnet, dass** sie einer der folgenden Formeln entspricht:
(SEQ ID Nr. 1) Arg-Asp-Cys-Arg-Arg
(SEQ ID Nr. 2) Asp-Cys-Arg-NH₂
(SEQ ID Nr. 3) Ser-Arg-Asp-Cys-Arg-Pro-Met-NH₂
(SEQ ID Nr. 4) Thr-Asp-Cys-Arg-Lys-Arg
(SEQ ID Nr. 5) Asp-Cys-Arg-Arg-Pro-Met-Gly-NH₂;
wobei die besagten Sequenzen der Formeln SEQ ID Nr. 1 bis SEQ ID Nr. 5 Substitutionen zwischen den folgenden Resten umfassen können:
- Ala, Val, Leu und Ile;
- Ser und Thr;
- Asp und Glu;
- Asn und Gln;
- Lys und Arg und
- Phe und Tyr.

2. Peptidverbindung nach Anspruch 1, **dadurch gekennzeichnet, dass** sie einer der folgenden Formeln entspricht:
(SEQ ID Nr. 1) Arg-Asp-Cys-Arg-Arg
(SEQ ID Nr. 2) Asp-Cys-Arg-NH₂
(SEQ ID Nr. 3) Ser-Arg-Asp-Cys-Arg-Pro-Met-NH₂
(SEQ ID Nr. 4) Thr-Asp-Cys-Arg-Lys-Arg
(SEQ ID Nr. 5) Asp-Cys-Arg-Arg-Pro-Met-Gly-NH₂.

3. Peptidverbindung nach einem der vorhergehenden Ansprüche, **dadurch gekennzeichnet, dass** sie als Arzneimittel verwendet wird.

4. Kosmetische Zusammensetzung, die die besagte Peptidverbindung nach einem der Ansprüche 1 oder 2 als Wirkstoff umfasst.

5. Kosmetische Zusammensetzung nach Anspruch 4, **dadurch gekennzeichnet, dass** sie in einer Form dargereicht wird, die auf eine topische Anwendung angepasst ist und die ein kosmetisch akzeptables Medium umfasst.

6. Zusammensetzung nach einem der Ansprüche 4 oder 5, **dadurch gekennzeichnet, dass** die besagte Peptidverbindung in der Zusammensetzung in einer Konzentration zwischen 0,0005 bis ungefähr 500 ppm und vorzugsweise in einer Konzentration zwischen 0,01 und 5 ppm vorliegt.

7. Zusammensetzung nach einem der Ansprüche 4 bis 6, **dadurch gekennzeichnet, dass** sie außerdem mindestens einen anderen Wirkstoff enthält, der die Wirkung der besagten Peptidverbindung begünstigt.

8. Zusammensetzung nach Anspruch 7, **dadurch gekennzeichnet, dass** der besagte Wirkstoff ein Mittel ist, das eine Aktivität im Gebiet von Aufhellern, wie Peeling-Mitteln; beruhigenden Mitteln, organischen oder anorganischen Sonnenschutzmitteln, Feuchtigkeitsspendern; anderen Depigmentierungsmitteln, Tyrosinasehemmern; Mitteln, die die Synthese von dermalen oder epidermalen Makromolekülen stimulieren und/oder ihren Abbau verhindern; Mitteln, die die Proliferation von Fibroblasten und/oder Keratinozyten stimulieren oder die Differenzierung von Keratinozyten stimulieren; Mitteln, die auf den Energiestoffwechsel von Zellen einwirken;
anderen depigmentierenden Peptiden, pflanzlichen Hydrolysaten, Anti-Aging-Mitteln sowie deren Gemischen aufweist.

9. Verwendung einer kosmetischen Zusammensetzung, die Folgendes umfasst:
- eine Peptidverbindung der folgenden allgemeinen Formel (I) :
Ri-X₁-X₂-Asp-Cys-Arg-X₃-X₄- (AA)ₚ -R₂.
in der
X₁ für eine Asparaginsäure, eine Glutaminsäure, ein Serin oder ein Threonin steht oder gleich null ist,
X₂ für ein Arginin, ein Leucin oder ein Isoleucin steht oder gleich null ist,
X₃ für ein Arginin oder ein Lysin steht oder gleich null ist,
X₄ für ein Arginin, ein Prolin, ein Histidin oder ein Lysin steht oder gleich null ist,
AA für eine beliebige Aminosäure, mit Ausnahme von Cystein, oder eines ihrer Derivate steht und p eine ganze Zahl zwischen 0 und 2 ist,
R₁ für die primäre Aminofunktion der N-terminalen Aminosäure steht, frei oder durch eine Schutzgruppe substituiert, die aus einer Acetylgruppe, einer Benzoylgruppe, einer Tosylgruppe oder einer Benzyloxycarbonylgruppe ausgewählt sein kann,
R₂ für die Hydroxylgruppe der Carboxylfunktion der C-terminalen Aminosäure steht, frei oder substituiert durch eine Schutzgruppe, die aus einer C₁- bis C₂₀-Alkylkette oder einer NH₂-, NHY- oder NYY-Gruppe, in der Y für eine C₁- bis C₄-Alkylkette steht, ausgewählt sein kann,
wobei die besagte Sequenz der allgemeinen Formel (I) aus 3 bis 9 Aminosäureresten besteht,
wobei die besagte Sequenz der allgemeinen Formel (I) Substitutionen der Aminosäuren X₁, X₂, X₃ und X₄ durch andere chemisch äquivalente Aminosäuren umfassen kann und
- ein kosmetisch akzeptables Medium,
zum Depigmentieren, Aufhellen und/oder Bleichen der Haut.

10. Verwendung einer kosmetischen Zusammensetzung, die die wie in einem der Ansprüche 1 oder 2 definierte besagte Peptidverbindung und ein kosmetisch akzeptables Medium umfasst, zum Depigmentieren, Aufhellen und/oder Bleichen der Haut.

11. Verwendung einer Zusammensetzung nach einem der Ansprüche 9 oder 10 zum Verhindern und/oder Behandeln von Hyperpigmentationsflecken, wie Melasma, Chloasma, aktinische Lentigo, Sonnen-Lentigo, Ephilide, erworbene Hyperpigmentationsflecken, Hyperpigmentationsflecken in Folge von Narben.

12. Verwendung einer Zusammensetzung nach einem der Ansprüche 9 oder 10 zum Behandeln und/oder Verhindern von Hautzeichen der Hyperpigmentationsart aufgrund von Lichtalterung.

13. Verfahren zur kosmetischen Behandlung, **dadurch gekennzeichnet, dass** eine Zusammensetzung, die eine wirksame Menge einer Peptidverbindung der folgenden allgemeinen Formel (I):
R₁-X₁-X₂-Asp-Cys-Arg-X₃-X₄- (AA)ₚ-R₂,
in der
X₁ für eine Asparaginsäure, eine Glutaminsäure, ein Serin oder ein Threonin steht oder gleich null ist,
X₂ für ein Arginin, ein Leucin oder ein Isoleucin steht oder gleich null ist,
X₃ für ein Arginin oder ein Lysin steht oder gleich null ist,
X₄ für ein Arginin, ein Prolin, ein Histidin oder ein Lysin steht oder gleich null ist,
AA für eine beliebige Aminosäure, mit Ausnahme von Cystein, oder eines ihrer Derivate steht und p eine ganze Zahl zwischen 0 und 2 ist,
R₁ für die primäre Aminofunktion der N-terminalen Aminosäure steht, frei oder durch eine Schutzgruppe substituiert, die aus einer Acetylgruppe, einer Benzoylgruppe, einer Tosylgruppe oder einer Benzyloxycarbonylgruppe ausgewählt sein kann,
R₂ für die Hydroxylgruppe der Carboxylfunktion der C-terminalen Aminosäure steht, frei oder substituiert durch eine Schutzgruppe, die aus einer C₁- bis C₂₀-Alkylkette oder einer NH₂-, NHY- oder NYY-Gruppe, in der Y für eine C₁- bis C₄-Alkylkette steht, ausgewählt sein kann,
wobei die besagte Sequenz der allgemeinen Formel (I) aus 3 bis 9 Aminosäureresten besteht,
wobei die besagte Sequenz der allgemeinen Formel (I) Substitutionen der Aminosäuren X₁ X₂, X₃ und X₄ durch andere chemisch äquivalente Aminosäuren umfassen kann;
als Wirkstoff umfasst, zum Depigmentieren, Aufhellen und/oder Bleichen der Haut topisch auf die zu behandelnde Haut oder die zu behandelnden Hautanhangsgebilde angewendet wird.

14. Verfahren zur kosmetischen Behandlung, **dadurch gekennzeichnet, dass** eine Zusammensetzung, die eine wirksame Menge einer wie in einem der Ansprüche 1 oder 2 definierten Peptidverbindung als Wirkstoff umfasst, zum Depigmentieren, Aufhellen und/oder Bleichen der Haut oder der Hautanhangsgebilde topisch auf die zu behandelnde Haut oder die zu behandelnden Hautanhangsgebilde angewendet wird.

15. Verfahren zur kosmetischen Behandlung, wie in einem der Ansprüche 13 oder 14 definiert, zum Verhindern und/oder Behandeln von Hautzeichen der Hyperpigmentationsart aufgrund von Lichtalterung.

## Claims

1. Peptide compound **characterised in that** it corresponds to one of the following formulas
(SEQ ID No. 1) Arg - Asp - Cys - Arg - Arg
(SEQ ID No. 2) Asp - Cys - Arg - NH2
(SEQ ID No. 3) Ser - Arg - Asp - Cys - Arg - Pro - Met - NH2
(SEQ ID No. 4) Thr - Asp - Cys - Arg - Lys - Arg
(SEQ ID No. 5) Asp - Cys - Arg - Arg - Pro - Met - Gly - NH2;
said sequences of formulas SEQ ID No. 1 to SEQ ID No. 5 possibly including substitutions between residues:
- Ala, Val, Leu and IIe;
- Ser and Thr;
- Asp and Glu;
- Asn and Gln;
- Lys and Arg; and
- Phe and Tyr.

2. Peptide compound according to claim 1, **characterised in that** it corresponds to one of the following formulas
(SEQ ID No. 1) Arg - Asp - Cys - Arg - Arg
(SEQ ID No. 2) Asp - Cys - Arg - NH₂
(SEQ ID No. 3) Ser - Arg - Asp - Cys - Arg - Pro - Met - NH2
(SEQ ID No. 4) Thr - Asp - Cys - Arg - Lys - Arg
(SEQ ID No. 5) Asp - Cys - Arg - Arg - Pro - Met - Gly - NH₂

3. Peptide compound according to one of the previous claims, **characterised in that** it is used as a medicine.

4. Cosmetic composition comprising said peptide compound according to one of claims 1 and 2 as active constituent.

5. Cosmetic composition according to claim 4, **characterised in that** it is in a form suitable for topical application comprising a cosmetically acceptable medium.

6. Composition according to one of claims 4 and 5, **characterised in that** said peptide compound is present in the composition with a concentration of between about 0.0005 and 500 ppm, and preferably with a concentration of between 0.01 and 5 ppm.

7. Composition according to any one of claims 4 to 6, **characterised in that** it also contains at least one other active constituent conducive to the action of said peptide compound.

8. Composition according to claim 7, **characterised in that** said active constituent is an agent with an activity in the domain of lightening agents such as peeling agents; salves, organic or inorganic photoprotection agents, moisturisers; other depigmentation agents, tyrosinase inhibitors; agents stimulating the synthesis of dermic or epidermic macromolecules and/or preventing their degradation, agents stimulating the proliferation of fibroblasts and/or keratinocytes or stimulating the differentiation of keratinocytes; agents acting on the energy metabolism of cells; other depigmentation peptides, plant hydrolysates, anti-aging agents, and mixes of them.

9. Use of a cosmetic composition comprising:
- a peptide compound with the following general formula (I) :
R₁ - X₁ - X₂ - Asp - Cys - Arg - X₃ - X₄ - (AA)ₚ- R₂
in which,
X₁ represents an aspartic acid, a glutamic acid, a serine, a threonine, or is equal to zero,
X₂ represents an arginine, a leucine, an isoleucine, or is equal to zero,
X₃ represents an arginine, a lycine, or is equal to zero, X₄ represents an arginine, a proline, a histidine, a lysine or is equal to zero,
AA represents any amino acid except for cysteine or one of its derivatives, and p is an integer number between 0 and 2,
R₁ represents the primary amine function of N-terminal amino acid, free or substituted by a protection group that can be chosen from among an acetyl group, a benzoyl group, a tosyl group or a benzylcarbonyl group,
R₂ represents the hydroxyl group of the carboxyl function of the C-terminal amino acid, free or substituted by a protection group that can be chosen from among a C₁ to C₂₀ alkyl chain, or an NH2, NHY or NYY group in which Y represents a C₁ to C₄ alkyl chain,
said sequence with general formula (I) being composed of 3 to 9 amino acid residuals,
said sequence with general formula (I) possibly including substitutions of amino acids X₁, X₂, X₃ and X₄ by other chemically equivalent amino acids; and
- a chemically acceptable medium,
to depigment, lighten and/or whiten the skin

10. Use of a cosmetic composition comprising said peptide compound as defined in one of claims 1 or 2 and a cosmetically acceptable medium to depigment, lighten and/or whiten the skin.

11. Use of a composition according to one of claims 9 or 10, to prevent and/or treat hyperpigmentation spots such as melasma, chloasma, actinic lentigo, solar lentigos, freckles, hyperpigmentation spots due to accidents, post-healing hyperpigmentation spots.

12. Use of a composition according to one of claims 9 or 10, to treat and/or prevent hyperpigmentation type cutaneous signs due to photo-aging.

13. Cosmetic treatment method **characterised in that** a composition comprising an effective quantity of peptide compound is applied topically to the skin or the nails or hair to be treated as active constituent, with the following general formula (I):
R₁-X₁-X₂-Asp-Cys-Arg-X₃-X₄-(AA)ₚ-R₂
in which,
X₁ represents an aspartic acid, a glutamic acid, a serine, a threonine, or is equal to zero,
X₂ represents an arginine, a leucine, an isoleucine, or is equal to zero,
X₃ represents an arginine, a lycine, or is equal to zero, X₄ represents an arginine, a proline, a histidine, a lysine or is equal to zero,
AA represents any amino acid except for cysteine or one of its derivatives, and p is an integer number between 0 and 2,
R₁ represents the primary amine function of N-terminal amino acid, free or substituted by a protection group that can be chosen from among an acetyl group, a benzoyl group, a tosyl group or a benzylcarbonyl group,
R₂ represents the hydroxyl group of the carboxyl function of the C-terminal amino acid, free or substituted by a protection group that can be chosen from among a C₁ to C₂₀ alkyl chain, or an NH2, NHY or NYY group in which Y represents a C₁ to C₄ alkyl chain,
said sequence with general formula (I) being composed of 3 to 9 amino acid residuals,
said sequence with general formula (I) possibly including substitutions of amino acids X₁, X₂, X₃ and X₄ by other chemically equivalent amino acids;
to depigment, lighten and/or whiten the skin

14. Cosmetic treatment method **characterised in that** a composition comprising an effective quantity of peptide compound as defined in one of claims 1 or 2 is applied topically to the skin or the nails or hair to be treated, as active constituent to depigment, lighten and/or whiten the skin or nails or hair

15. Cosmetic treatment method as defined in one of claims 13 or 14, to prevent and/or treat hyperpigmentation type cutaneous signs due to photo-aging.
